# EUROPEAN PATENT APPLICATION

(11) **EP 3 492 134 A1**
(43) Date of publication of application: **05.06.2019**
(21) Application number: 18208839.3
(22) Date of filing: 28.11.2018
(51) Int. Cl.: A61M 25/02

(54) **CATHETER SECUREMENT DEVICE WITH AT LEAST ONE VERTICAL SLIT**

(30) Priority: 30.11.2017 US 201762592710 P
(71) Applicant: Tidi Products, LLC, Neenah, WI 54956 (US)
(72) Inventor: Parkhurst, Arthur, Ocala, FL 34472 (US)
(74) Representative: Barker Brettell LLP

(57) **Abstract**

A catheter securement device (20) for securing a catheter (26) with a hub (28) and at least one tube member (26). The catheter securement device may include a flexible, thin, sheet-like base member (32) and a releasable, flexible, thin, sheet-like retention member (34). The retention member may include at least one vertical slit (44) that extends substantially perpendicular from the distal edge (40). The tube member may extend through the vertical slit. Also, a hole (46) may be located between the distal edge and the proximal edge at the vertical slit. Additional vertical slits (48, 50) may be located about the retention member to accommodate additional tube members. The slits allow the tube members to extend from the retention member substantially midway between a distal edge and a proximal edge of the retention member.

## Description

### CROSS-REFERENCE TO RELATED APPLICATION

The present application claims the benefit of U.S. Provisional Patent Application Serial No. 62/592,710 filed on November 30, 2017 and titled *Catheter Securement Device with at Least One Vertical Slit*, the entirety of which is incorporated by reference herein.

### BACKGROUND OF THE INVENTION

### 1. Field of the Invention

The present invention relates in general to the field of medical devices referred to as "catheter securement devices". More particularly, the present invention relates to a catheter securement device with superior holding capabilities. Specifically, a preferred embodiment of the present invention relates to a catheter securement device having at least one vertical slit, which allows the catheter securement device to remain secured to a patient's skin despite significant loads on the catheter and associated components.

### 2. Discussion of the Related Art

A variety of catheter securement devices is known to those skilled in the art. For instance, many types of catheter securement devices are made of a flexible bandage-type material that is secured to a patient's skin. Oftentimes, these devices have a base member that is secured to the skin and a retention member that can be releasably attached to the base member. As a result, the retention member can repeatedly be opened and closed to allow for adjustment of the catheter and associated components. By way of example, a number of catheter securement devices are shown in U.S. Patent Nos. D608,887; D616,091; D616,983; D625,002; D715,927; D715,928; D780,914; 8,251,957; 8,500,698; and 9,248,259, all of which are incorporated herein by reference. While the catheter securement devices shown in these patents successfully secure a catheter in place, alternative catheter securement devices that are capable of withstanding greater forces prior to failure are desired.

One alternative catheter securement device that is capable of withstanding greater forces prior to failure in comparison to traditional catheter securement devices is shown in U.S. Patent No. 9,358,366 and U.S. Patent Appl. Publ. No. 2016/0331934, both of which are incorporated herein by reference. The catheter securement device in these references includes a retention member that is smaller than the base member, resulting in an offset between the distal edge of the base member and the retention member. As a result of this offset, any forces applied to tubes associated with the catheter will be distributed down through the middle of the base member. In contrast, many prior art catheter securement devices have a retention member that is substantially the same size as the base member. This results in a distal edge of the base member that is substantially flush with the distal edge of the retention member. Therefore, when forces are applied to tubes associated with the catheter, the distal edge of the base member can sometimes be peeled off somewhat easily. Because the forces are distributed down through the middle of the base member in the improved device instead of at the edge of the base member, which occurs with traditional catheter securement devices, this improved catheter securement device is capable of withstanding greater forces prior to failure. However, additional alternative designs capable of withstanding significant forces prior to failure are still desired.

What is therefore needed is another catheter securement device specifically configured to withstand forces applied to the catheter and related devices prior to failure.

### SUMMARY AND OBJECTS OF THE INVENTION

By way of summary, the present invention is directed to a catheter securement device that is mounted to the skin of a patient to secure a catheter including a hub and a tube member. In accordance with a first aspect of the invention, the catheter securement device includes a flexible, thin, sheet-like base member and a releasable, flexible, thin, sheet-like retention member. The base member may have a proximal edge, a distal edge opposite the proximal edge, a first end, a second end opposite the first end, a top side, and a bottom side. Additionally, a release liner may be releasably mounted to the bottom side of the base member.

The retention member may have a first end, a second end opposite the first end, a distal edge, a proximal edge opposite the distal edge, a top side, a bottom side, and at least one vertical slit extending substantially perpendicular from the distal edge. Additionally, one of the first end and the second end may be a fixed end and the other of the first end and the second end may be a releasable end. When in use, the hub is secured beneath the retention member. Additionally, the tube member may extend through a first vertical slit, which may be located substantially midway between the first end and the second end. The retention member may also have a hole located between the distal edge and the proximal edge where the hole allows the retention member to accommodate the catheter with a plurality of tube members. For instance, the first slit may terminate at the hole. Additionally, the retention member may have a second vertical slit and a third vertical slit. Both of these slits are configured to accommodate additional tube members, such as a second tube member and a third tube member.

In accordance with another aspect of the invention, the catheter securement device may have a focus area that results from forces that are applied to one of the tube members associated with the catheter. Preferably, the focus area is located substantially midway between the distal edge and the proximal edge. This focus area could result from upward forces that are applied to the tube members or lateral forces that are applied to the tube members.

In accordance with yet another aspect of the invention, a hook-and-loop fastener is used to releasably attach the retention member to the base member. For instance, one of a hook-and-loop fastener may be mounted to a top side of the base member and the other of a hook-and-loop fastener may be mounted to the bottom side of the retention member. Both or either of the hook-and-loop fasteners may be impregnated with an adhesive for enhanced gripping characteristics.

In accordance with another aspect of the invention, a method of using a catheter securement device is provided. First, a catheter securement device as described herein is provided. Next, a release liner is removed from a bottom side of the base member to expose adhesive. The bottom side of the base member is then affixed to the patient's skin. Next, a hub that is associated with a catheter and at least one tube is located directly adjacent to a top side of the base member. Thereafter, the retention member is releasably-affixed to the top side of the base member to secure the hub and catheter in place. Also, at least one tube may be located through the at least one vertical slit. Further still, the hub may be located beneath the hole and a plurality of tubes may be threaded through the hole. Additional tubes may similarly be threaded through additional vertical slits, for instance, first, second, and third tubes threaded through first, second, and third slits.

These and other aspects and objects of the present invention will be better appreciated and understood when considered in conjunction with the following description and the accompanying drawings. It should be understood, however, that the following description, while indicating preferred embodiments of the present invention, is given by way of illustration and not of limitation. Many changes and modifications may be made within the scope of the present invention without departing from the spirit thereof, and the invention includes all such modifications.

### BRIEF DESCRIPTION OF THE DRAWINGS

A clear conception of the advantages and features constituting the present invention, and of the construction and operation of typical mechanisms provided with the present invention, will become more readily apparent by referring to the exemplary, and therefore non-limiting, embodiments illustrated in the drawings accompanying and forming a part of this specification, wherein like reference numerals designate the same elements in the several views, and in which:
FIG. 1 illustrates the inventive catheter securement device having a single vertical slit and a hole formed in a retention member;
FIG. 2 illustrates the inventive catheter securement device of FIG. 1 with a catheter installed on the catheter securement device;
FIG. 3 illustrates the inventive catheter securement device of FIGS. 1 and 2 where at least one tube member is being pulled;
FIG. 4 illustrates the inventive catheter securement device of FIGS. 1-3 where at least one tube member is being pulled upwardly;
FIG. 5 illustrates the inventive catheter securement device of FIGS. 1-4 where at least one tube member is being further pulled upwardly;
FIG. 6 illustrates the inventive catheter securement device having three vertical slits and a hole formed in a retention member;
FIG. 7 illustrates the inventive catheter securement device having three vertical slits formed in a retention member;
FIG. 8 illustrates a longitudinal view of the device of FIGS. 2-5 where at least one tube is being pulled upwardly;
FIG. 9 illustrates a longitudinal view of the device of FIG. 6 where at least one tube is being pulled upwardly;
FIG. 10 illustrates the inventive catheter securement device of FIGS. 1-5 where the retention member is in an open position; and
FIG. 11 illustrates the inventive catheter securement device of FIGS. 1-5 and 10 where a catheter and hub are located relative to the base member.

In describing the preferred embodiment of the invention which is illustrated in the drawings, specific terminology will be resorted to for the sake of clarity. However, it is not intended that the invention be limited to the specific terms so selected and it is to be understood that each specific term includes all technical equivalents which operate in a similar manner to accomplish a similar purpose. For example, the word connected, attached, or terms similar thereto are often used. They are not limited to direct connection but include connection through other elements where such connection is recognized as being equivalent by those skilled in the art.

### DESCRIPTION OF PREFERRED EMBODIMENTS

The present invention and the various features and advantageous details thereof are explained more fully with reference to the non-limiting embodiments described in detail in the following description.

### 1. System Overview

The inventive catheter securement device includes at least one vertical slit that is designed to accommodate at least one line or tube member associated with a catheter. The device may have a bottom made of a soft material like a bandage. Additionally, the vertical slit may terminate in a hole, which could be used for a Foley catheter with a multiple-lumen hub. The catheter securement device may have additional vertical slits and/or holes for additional lumens. As a result of the slit(s) and/or the hole(s), forces applied to the lumens do not result in the hub pulling on the edge of the catheter securement device, which would cause the device to fail more easily. Such an arrangement allows for a catheter securement device capable of securing hubs of a number of different sizes. Additionally, an adhesive strip may extend along the catheter securement device. Alternatively, a hook-and-loop fastener can be used.

### 2. Detailed Description of Preferred Embodiments

In general, the invention is a catheter securement device 20 that adheres to the skin 22 of a patient having an epidural catheter 26 or similar small flexible tube member inserted percutaneously. The securement device 20 acts to secure and restrain the catheter tube 26 or housing to prevent or reduce movement near the insertion site or accidental dislodgement of the catheter 26. The securement device 20, in general, comprises a flexible, thin, sheet-like base member 32 capable of adhering to the skin 22 of the patient and a releasable, flexible, thin, sheet-like retention member 34 to secure the catheter 26 to the base member 32.

As used herein, the term "catheter" shall include a tube member alone or multiple tubes in combination with a catheter hub 28 or housing member. The term "proximal" shall refer to the direction from the catheter securement device 20 toward the catheter insertion site and the term "distal" shall refer to the direction away from the catheter securement device 20 opposite from the insertion site. The term "proximal tube" shall refer to the tube extending between the insertion site and the securement site and the term "distal tube" shall refer to a tube extending from the securement device 20 on the side opposite that of the insertion site. The term "proximal edge" shall refer to the side of the catheter securement device 20 facing toward the insertion site and the term "distal edge" shall refer to the side of the securement device 20 facing away from the insertion site. The position of the catheter 26 relative to the securement device 20 when the device 20 is in use shall generally define the "axis" or the "axial direction" of the catheter 26, with the term "transverse" defining the direction generally parallel to the axial direction, i.e., the width of the device 20, and the term "longitudinal" defining the direction perpendicular to the transverse direction. The term "transverse midline" shall define a line located generally parallel to and in approximate vertical alignment with the catheter axis when the device 20 is in use, which is preferably located at the true dimensionally-centered midline of the base member 32 and retention member 34. The terms "midportion" and "interior" shall refer to a location away from the outer edges of the base member 32. The term "uplift focus area" shall refer to a point or small area where uplift forces 58, resulting from pulling a distal tube in the direction perpendicular, i.e. vertical, to the skin 22 of the patient, are initially concentrated on or transferred to the base member 32 of the catheter securement device 20.

Turning now to the figures, the inventive catheter securement device 20 includes a base member 32 and a retention member 34. The base member 32 may be flexible, thin, and sheet-like with an adhesive backing on a bottom side 64 that is covered by a release layer. Once the release layer has been removed to expose the adhesive backing, the base member 32 can adhere directly to a patient's skin 22. As shown, the base member 32 is generally elongated and preferably in a "dog bone" configuration with a proximal edge 82 and a distal edge 80, both of which are concave. Additionally, the base member 32 has a first end 76 and a second end 78 that are both rounded. A transverse midline 84 extends from the proximal edge 82 to the distal edge 80 midway between the first end 76 and the second end 78. The base member 32 also has a top side.

Similarly, the retention member 34 may be flexible, thin, and sheet-like. The retention member 34 may also be generally elongated and preferably in a "dog bone" configuration with a proximal edge 42 and a distal edge 40, both of which are concave. The retention member 34 also has a first end 36 and second end 38 that are rounded. Further still, the retention member 34 may have a top side 66 and a bottom side 68 opposite the top side 66. In one embodiment, the first end 36 may be a fixed end 70 and the second end 38 is a releasable, free end 72. Alternatively, both ends 36, 38 could be releasable. Additionally, a transverse midline 86 may extend from the proximal edge 42 to the distal edge 40 across the retention member 34 midway between the first end 36 and the second end 38. As shown, the retention member 34 is substantially the same size as the base member 32, although the retention member 34 could also be smaller than the base member 32.

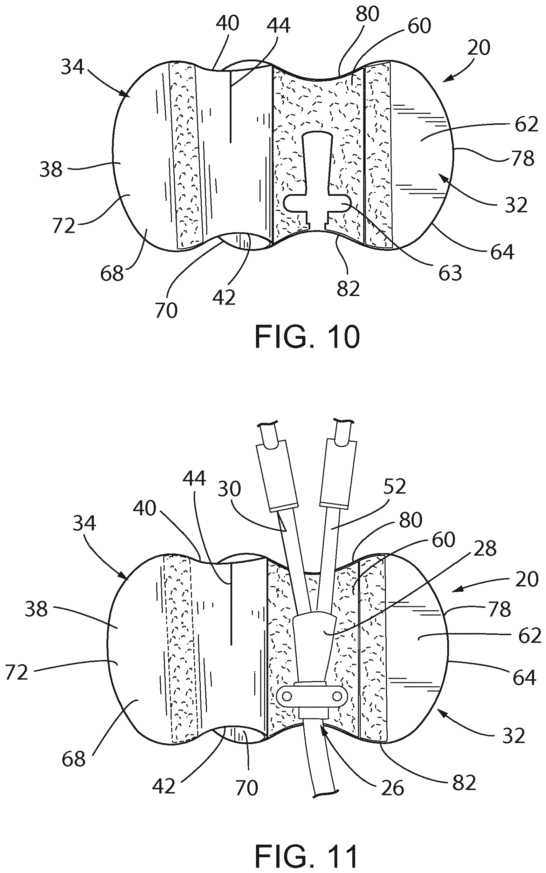

The retention member 34 can be releasably attached to the base member 32 using any number of fastening mechanisms 60, including, for instance, a hook-and-loop fastener, pressure-sensitive adhesive, or the like. Preferably, the fastening mechanism 60 securely retains the retention member 34 onto the base member 32 when the retention member 34 is pulled tautly across the catheter 26 and hub 28 to preclude the catheter 26 or the hub 28 from undesired movement. When this occurs, the transverse midlines 84, 86 overlap so as to be generally vertically aligned when the retention member 34 is closed over the catheter 26 and hub 28. Similarly, the fastener mechanism 60 hook-and-loop fastener may also be impregnated with adhesive to improve overall strength of the connection between the hook-and-loop fasteners. Further, the hook-and-loop fastener may have a specific configuration to help a user locate the catheter 26 and/or hub 28 relative to the base member 32. For instance, as shown in FIGS. 10 and 11, the fastener mechanism 60 may be a hook-and-loop fastener located on a top side 62 of the base member 32 and a bottom side 68 of the retention member 34, with the hook-and-loop fastener of the base member 32 having a cutout 63 that mirrors the outline of the hub 28. In this way, once the base member 32 is secured to the patient's skin 22, the hub 28 can be located to align with the cutout. Preferably, the hub 28 and catheter 15 align with the transverse midline of the catheter securement device 20.

The retention member 34 may have a number of slits, openings, and the like to accommodate catheters 26 and associated hubs 28. These slits, openings, and the like allow the catheter securement device 20 to remain attached to the patient's skin 22 despite having significant loads applied to the catheter 26, the hub 28, or other members associated therewith. While certain embodiments will be described below, it should be noted that additional slits and openings may be formed in the retention member 34, as would be appreciated by one skilled in the art.

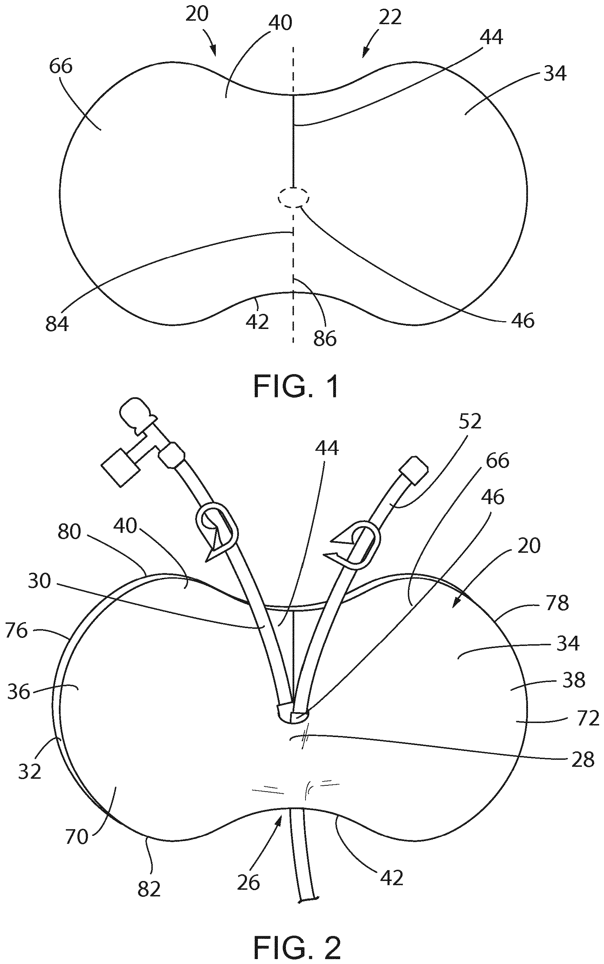

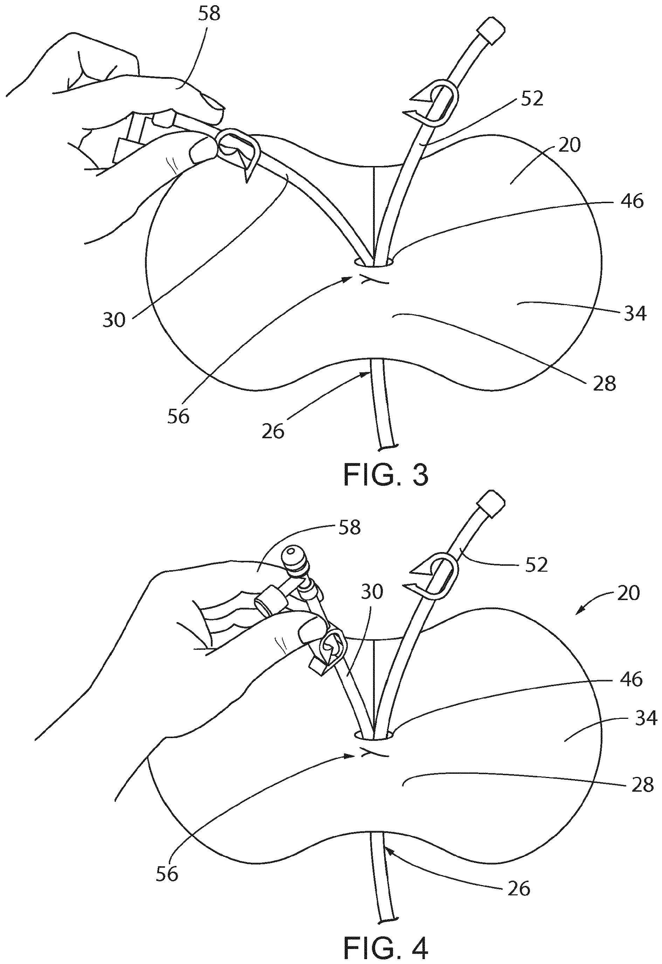

A first embodiment of the inventive catheter securement device 20 is shown in FIGS. 1-5. This embodiment features a single vertical slit 44 that is formed in the retention member 34. Additionally, a hole 46 or opening is located where the slit 44 terminates. The catheter securement device 20 is configured such that the catheter 26 and associated hub 28 are secured between the base member 32 and the retention member 34. More specifically, as can be seen in FIG. 2, the hub is located between the base member 32 and the retention member 34 along the bottom half of the catheter securement device 20. The slit 44 allows at least one tube member 30 to be threaded through the retention member 34. A distal edge of the hub 28 may extend slightly past the hole 46. In this way, the hub 28 can be secured in place relative to the base member 32 and the patient's skin.

Use of the catheter securement device 20 of FIGS. 1-5 will now be described. First, the catheter securement device 20 is mounted to the patient's skin 22. This may occur by removing release liners to expose adhesive that can hold the catheter securement device 20 to the patient's skin 22. Next, at least one of the ends 36, 38 of the retention member 34 is moved away from the base member 32 to an open position, as shown in FIGS. 10 and 11. Alternatively, where the retention member 34 has two releasable ends, the base member 32 is simply installed to the patient's skin 22. Thereafter, the catheter 26 and hub 28 are located adjacent to the base member 32. As described above, the base member 32 may have a fastener mechanism 60 such as a hook-and-loop fastener with a cutout 63 of substantially the same dimensions as the hub 28. This allows for quick placement of the hub 28 in the appropriate position. Alternatively, the hub 28 may be pressed against a fastener, such as an adhesive or a hook-and-loop fastener of the base member 32, which would initially secure the hub 28 in place. Alternatively still, the hub 28 may simply be rested upon the base member 32 until the retention member 34 is secured to the base member 32. The retention member 34 is then moved to a closed position shown in FIGS. 1-5 where the retention member 34 is secured to the base member 32, with the catheter 26 and hub 28 secured therebetween, as seen in FIGS. 2-5. Thereafter, the at least one tube 30 is threaded through the slit 44.

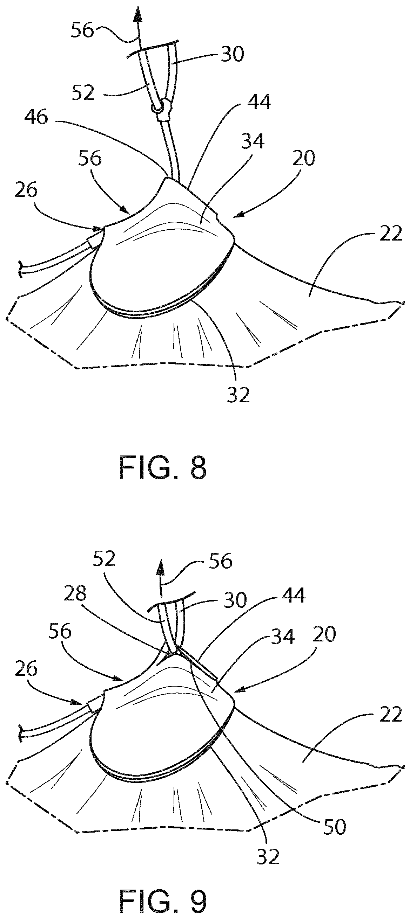

Because the tube 30 extends from the middle of the catheter securement device 20, any forces to displace the tube 30, including uplift, lateral, and any other forces, will be transmitted to the middle of the catheter securement device 20 and, in turn, to the middle of the base member 32. This results in an uplift focus area 56 that occurs at or adjacent to the hole 46 formed in the retention member 34, as can be seen in FIG. 8. Thus, the catheter securement device 20 shown in the figures can accommodate significantly greater forces than if the uplift focus area occurred at the distal edge of the base member, as is common in the prior art, which could cause the base member to peel back at the distal edge and fail with lesser forces.

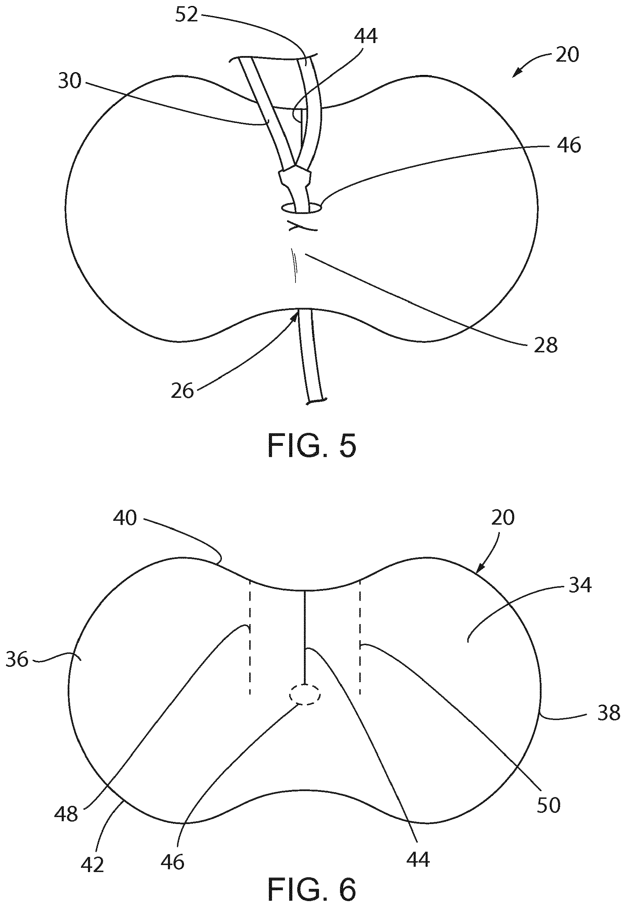

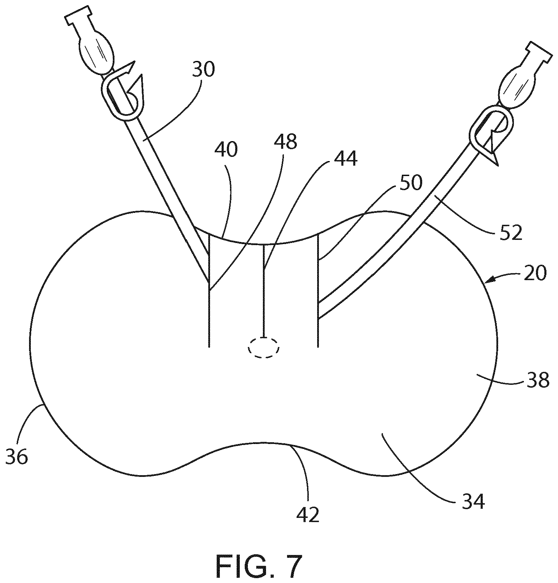

Turning next to FIGS. 6 and 7, a second embodiment of the inventive catheter securement device 20 is shown. Here, the catheter securement device may have additional vertical slits. For instance, as shown, this catheter securement device 20 includes a first vertical slit 44, a second vertical slit 48, and a third vertical slit 50 formed in the retention member 34. As shown in FIG. 6, the retention member 34 may also include a hole 46 located where the first slit 44 terminates. This embodiment is capable of securing a catheter 26 and hub 28 having multiple tubes members 30, 52 about the multiple slits 44, 48, 50. Again, the catheter securement device 20 is configured such that the catheter 26 and associated hub 28 are secured between the base member 32 and the retention member 34. More specifically, as can be seen in FIG. 7, the hub 28 is located between the base member 32 and the retention member 34 along the bottom half of the catheter securement device 20. The slits 44, 48, 50 allow the tube members 30, 52 to be threaded through the retention member 34. A distal edge of the hub 28 may extend slightly past the hole 46. In this way, the hub 28 can be secured in place.

Use of the catheter securement device 20 of FIGS. 6 and 7 will now be described. Initially, the catheter securement device 20 is mounted to the patient's skin 22. Again, this may occur by removing release liners to expose adhesive that can hold the catheter securement device 20 to the patient's skin 22. Next, at least one of the ends 36, 38 of the retention member 34 is moved away from the base member 32 to an open position, similar to what is shown in FIGS. 10 and 11. Thereafter, the catheter 26 and hub 28 are located adjacent to the base member 32. As described above, the base member 32 may have a hook-and-loop fastener with a cutout of substantially the same dimensions as the hub 28. This allows for quick placement of the hub 28 in the appropriate position. Alternatively, the hub 28 may be pressed against a fastener, such as an adhesive or a hook-and-loop fastener of the base member 32, which would initially secure the hub 28 in place. Alternatively still, the hub 28 may simply be rested upon the base member 32 until the retention member 34 is secured to the base member 32. The retention member 34 is then moved to a closed position, as shown in FIG. 7, where the retention member 34 is secured to the base member 32, with the catheter 26 and hub 28 secured therebetween as seen in FIG. 7. While the retention member 34 is moved to the closed position, each of the tube members 30, 52 is threaded through any of the slits 44, 48, 50.

Because the tubes 30, 52 extend from the middle of the catheter securement device 20, any forces to displace the tube 30, including uplift, lateral, and any other forces, will be transmitted to the middle of the catheter securement device 20 and, in turn, to the middle of the base member 32. This results in an uplift focus area 56 that occurs at, or adjacent to, the hole 46 formed in the retention member 34. Thus, the catheter securement device 20 shown in the figures can accommodate significantly greater forces in multiple directions to the tubes 30, 52 than if the uplift focus area found in the prior art, which oftentimes occurred at the distal edge of the base member, which could cause the base member to peel back at the distal edge and fail with lesser forces.

By having slit(s) 44, 48, 50 and/or hole(s) 46 formed in retention member 34, the uplift focus area 56 that occurs when forces are applied to the catheter 26, hub 28, or related components is shifted into a midportion of interior of the base member 32, as can best be appreciated with review of FIGS. 8 and 9. In turn, the forces are dispersed across the entire footprint of the base member 32, resulting in a tenting or coning effect on the base member 32 and the underlying skin 22. This reduces the likelihood of detachment of the base member 32 since a much greater force is required to separate the base member 32 away from the skin 22 in comparison to the force required to peel the base member 32 from the skin at the distal edge 80.

All the disclosed embodiments are useful in conjunction with the securement of catheters, hubs, tubs, and other devices that need to be secured relative to a patient during medical procedures, or any other situations when a product needs to be secured close to a patient's skin. There are virtually innumerable uses for the present invention, all of which need not be detailed here. All the disclosed embodiments can be practiced without undue experimentation.

Although the best mode contemplated by the inventors of carrying out the present invention is disclosed above, practice of the present invention is not limited thereto. It will be manifest that various additions, modifications, and rearrangements of the features of the present invention may be made without deviating from the spirit and scope of the underlying inventive concept.

In addition, the individual components need not be fabricated from the disclosed materials but could be fabricated from virtually any suitable materials. Similarly, a variety of different attachment materials could be used to secure the various components, including the base member and the retention member together.

Moreover, the individual components need not be formed in the disclosed shapes, or assembled in the disclosed configuration, but could be provided in virtually any shape, and assembled in virtually any configuration. Furthermore, all the disclosed features of each disclosed embodiment can be combined with, or substituted for, the disclosed features of every other disclosed embodiment except where such features are mutually exclusive.

It is intended that the appended claims cover all such additions, modifications, and rearrangements. Expedient embodiments of the present invention are differentiated by the appended claims.

## Claims

1. A catheter securement device that is mounted to the skin of a patient to secure a catheter comprising a hub and a tube member, the device comprising:
a flexible, thin, sheet-like base member;
a releasable, flexible, thin, sheet-like retention member having a first end, a second end, a distal edge, a proximal edge, and at least one vertical slit extending substantially perpendicular from the distal edge;
wherein the hub is secured beneath the retention member; and
wherein the tube member extends through a first vertical slit.

2. The catheter securement device of claim 1, further comprising a hole located between the distal edge and the proximal edge.

3. The catheter securement device of claim 2, wherein the first slit terminates at the hole, and wherein the hole allows the retention member to accommodate the catheter with a plurality of tube members.

4. The catheter securement device of claim 1, wherein the at least one vertical slit includes the first vertical slit and a second vertical slit.

5. The catheter securement device of claim 4, wherein the first vertical slit accommodates a first tube member and the second vertical slit accommodates a second tube member.

6. The catheter securement device of claim 1, wherein the at least one vertical slit includes the first vertical slit, a second vertical slit, and a third vertical slit.

7. The catheter securement device of claim 6, wherein the first vertical slit accommodates a first tube member, the second vertical slit accommodates a second tube member, and the third vertical slit accommodates a third tube member.

8. The catheter securement device of claim 7, wherein an uplift focus area resulting from forces applied to one of the first tube member, the second tube member, and the third tube member is located substantially midway between the distal edge and the proximal edge.

9. The catheter securement device of claim 8, wherein the uplift focus area results from upward forces applied to one of the first tube member, the second tube member, and the third tube member.

10. The catheter securement device of claim 8, wherein the uplift focus area results from lateral forces applied to one of the first tube member, the second tube member, and the third tube member.

11. The catheter securement device of claim 6, further comprising at least one of the following:
i) a hole located between the distal edge and the proximal edge, wherein the first vertical slit terminates at the hole; and
ii) wherein the first vertical slit is substantially midway between the first end and the second end.

12. The catheter securement device of claim 1, further comprising at least one of the following:
i) one of a hook-and-loop fastener mounted to a top side of the base member; and the other of a hook-and-loop fastener mounted to a bottom side of the retention member; wherein the hook-and-loop fasteners are impregnated with an adhesive; and ii) wherein the retention member further comprises a fixed end and a releasable end.

13. A method of using a catheter securement device comprising the steps of:
providing a catheter securement device with:
a flexible, thin, sheet-like base member with a bottom side and a top side;
a releasable, flexible, thin, sheet-like retention member having a first end, a second end, a distal edge, a proximal edge, and at least one vertical slit extending substantially perpendicular from the distal edge; and
at least one release liner mounted to the bottom side of the base member;
removing the at least one release liner from the bottom side of the base member;
affixing the bottom side of the base member to a patient's skin;
locating a hub associated with a catheter and at least one tube directly adjacent to the top side; and
releasably-affixing the retention member to the top side of the base member to secure the hub and the catheter in place.

14. The method of claim 13, further comprising one or more of the following:
i) further comprising the step of locating the at least one tube through the at least one vertical slit,
ii) wherein the retention member further comprises a hole located at the end of the at least one vertical slit, further comprising the steps of:
locating the hub beneath the hole; and
threading a plurality of tubes through the hole; and iii) wherein the retention member has a first vertical slit, a second vertical slit, and a third vertical slit, further comprising the steps of:
locating the hub beneath the retention member, wherein the hub has a first tube, a second tube, and a third tube extending therefrom;
threading the first tube through the first vertical slit;
threading the second tube through the second vertical slit; and
threading the third tube through the third vertical slit.

15. A catheter securement device that is mounted to the skin of a patient to secure a catheter comprising a hub and a tube member, the device comprising:
a flexible, thin, sheet-like base member with a proximal edge, a distal edge opposite the proximal edge, a first end, a second end opposite the first end, a top side, and a bottom side;
a releasable, flexible, thin, sheet-like retention member having a proximal edge, a distal edge opposite the proximal edge, a first end, a second end opposite the first end, a top side, a bottom side, a first vertical slit extending substantially perpendicular from the distal edge toward the proximal edge, a second vertical slit extending substantially perpendicular from the distal edge toward the proximal edge, a third vertical slit extending substantially perpendicular from the distal edge toward the proximal edge, and a hole located where the first vertical slit terminates;
a first of a hook-and-loop fastener impregnated with an adhesive mounted to the top side of the base member;
a second of a hook-and-loop fastener impregnated with an adhesive mounted to the bottom side of the retention member; and
at least one release liner releasably mounted to the bottom side of the base member;
wherein the hub is secured beneath the hole; and
wherein a first tube member extends through the first vertical slit, a second tube member extends through the second vertical slit, and a third tube member extends through the third vertical slit.
